# EUROPEAN PATENT APPLICATION

(11) **EP 3 501 543 A1**
(43) Date of publication of application: **26.06.2019**
(21) Application number: 17208532.6
(22) Date of filing: 19.12.2017
(51) Int. Cl.: A61K 47/34, A61K 31/565, B33Y 10/00

(54) **METHOD FOR MANUFACTURING VAGINAL DRUG DELIVERY SYSTEMS USING A THREE DIMENSIONAL PRINTER**

(71) Applicant: Delim Cosmetics & Pharma S.r.l., 20124 Milano (IT); Strusi, Orazio Luca, 08207 Sabadell (ES)
(72) Inventor: RONCHI, Celestino, 20124 MILANO (IT); RONCHI, Federica, 20124 MILANO (IT); STRUSI, Orazio Luca, 08207 SABADELL (ES); ORLANDINI, Milena, 08860 CASTELLDEFELS (Barcelona) (ES)
(74) Representative: Bianchetti Bracco Minoja S.r.l.

(57) **Abstract**

The present invention relates to a method for manufacturing a controlled release vaginal ring using a three-dimensional (3D) printer, said vaginal ring comprising:
a) a core, said core consisting of a composition comprising one or more thermoplastic polyurethanes and one or more active pharmaceutical ingredients dissolved or dispersed in said core,
b) a membrane, substantially or completely surrounding said core, said membrane consisting of a composition comprising one or more thermoplastic polyurethanes.

## Description

The present invention relates to a method for manufacturing a controlled release vaginal ring by using a three-dimensional (3D) printer and to the product obtained by said method.

Vaginal drug delivery systems that release a drug steadily over a long period such as 21 days, as in the classic contraceptives, may be matrix systems or reservoir systems.

Matrix systems are toroidal rings formed by a polymer that includes the active principle in suspension or in solution. The release of the active principle is controlled by the ability of the active principle to free itself from the polymer.

Reservoir systems are toroidal rings consisting of a core, where the active principle is dissolved or suspended, and an outer membrane, covering the core, that controls the release of the active principle.

This route of administration of a drug prevents enteric gastrointestinal absorption and enterohepatic passage by increasing the bioavailability of the drug when compared with oral administration.

The contraceptive vaginal ring may contain only one active principle or a combination of active principles.

Examples of contraceptive vaginal ring containing only one active principle are progestin - vaginal ring which may contain progesterone, levonorgestrel or nestorone.

Vaginal rings containing an estrogen-progestogen combination are widely used for 3 weeks 'in' and 1 week 'out' treatments. NuvaRing® is a vaginal ring in ethylvinyl acetate which releases 120 mcg /die of Etonogestrel (active metabolite of desogestrel) and 15 mcg/die of ethinyl estradiol.

Vaginal rings are also used in hormone replacement therapy (TOS) for postmenopausal women.

Currently there are two products on the market: Estring of Pfizer and Femring of Warner Chilcott containing estradiol and estradiol acetate respectively.

Vaginal rings can also be used for the administration of active principles for different pathologies, such as glyminox as contraceptive and for the prevention of sexually transmitted diseases, such as terbutaline for dysmenorrhoea and endometriosis, demegen for the prevention of sexually transmitted diseases, lidocaine for cervical anesthesia, oxybutinin for overactive bladder, tenofovir for the prevention of HIV transmission and antibody III-174 for the prevention and treatment of herpes simplex virus 2.

The polymers used in vaginal rings are silicone polymers and ethylene vinyl acetate polymers. The vaginal ring NuvaRing currently present in the market is made of ethylene vinyl acetate polymers.

In both cases, in order to ensure a constant release over time, which varies from 21 days to 90 days, the rings currently marketed (reservoir systems) have toroidal and coaxial shape, consisting of a core, comprising a polymer and an active principle, and a membrane that covers the core, made up of a polymer that controls the release of the active ingredient continuously over time (zero order release).

The production of said reservoir systems requires high-tech plants and important investments both for development and for commercial production.

Recently, Welsh at al presented at HIV R4P 2016 a paper entitled "3D printing of microbial vaginal rings: a proof-of-concept study" disclosing a vaginal ring produced using a 3D printer (Fused Deposition Modeling). The vaginal ring is a matrix system (a vaginal ring of toroidal shape without an external membrane controlling the release of the active principle) consisting of a polymer mixture of polylactic acid (PLA) and a thermoplastic polyurethane in a 1: 4 ratio, containing davirapin and / or levonogestrel as active ingredients.

WO2016/025388 discloses a method for manufacturing a bioactive implant comprising the steps of:
a. forming a mixture of a bioactive agent with a polymer stock material;
b. heating the mixture to an approximate melt flow temperature of the polymer stock material;
c. forming the mixture into the shape of the implant
wherein the step of forming the mixture into the shape of an implant comprises 3D printing of the mixture.

The polymer stock is a biodegradable polymer such as poly(methyl methacrylate), acrylonitrile butadiene styrene, polycarbonate, polylactide, polyglycolide, polycaprolactone, polyanhydride, polyorthocarbonate, polyvinylpyrrolidone and chitosan.

The bioactive implant has a honeycombed structure having a void content of at least 5%.

WO2017/054433 discloses a polyurethane composition, consisting of two types of polyurethane, having an adjustable elastic modulus. The polyurethane composition is used for preparing medical implants such as implantable devices, implantable prostheses, contact prostheses, stents, interventional catheters and organ auxiliary devices. In particular, the document discloses a degradable scaffold complex comprising a polyurethane composition and a degradable metal material. A biodegradable scaffold can be manufactured by using a 3D printer equipped with two feeding devices, one feeding device is added with magnesium alloy powder and the other feeding device is added with biodegradable medical polyurethane solution.

In the state of art, 3D printing, based on FDM technology, was used in the field of implant and topical application for manufacturing devices consisting of one polymer or a combination of more polymers which were extruded from one nozzle to give a matrix object.

The known 3D printing technology, wherein just one material can be extruded per time does not allow manufacturing in one step vaginal rings with reservoir technology, as the rings actually available on the market. For example, first the core should be manufactured and removed from the printer. The membrane should be manufactured in a second printer and then assembled with the core. Alternatively, half membrane could be printed and two half parts of the membrane could then be assembled with the core. This process is time consuming, expensive and semi-manual, making the 3D printing not competitive compared with the present method of manufacturing.

The most common process of obtaining vaginal rings is hot melt extrusion, where the polymers, alone or mixed with drugs, are melted and forced by extrusion screws to pass through a die. After leaving the die, the obtained coaxial fiber is cooled and cut, the obtained fragments are shaped as rings by gluing both ends with an adequate pharmaceutical adhesive or by injection molding.

This manufacturing process is time-consuming, it needs tailored equipments for extruding the polymers, cooling down the coaxial fiber that comes out from the twin extruder, a machine for cutting the fiber in short segments which are fitted in a machine for its sealing.

The present invention provides a one step process for manufacturing vaginal rings with reservoir technology by using a 3D printer, which makes the manufacture of the rings cheaper, faster and flexible in terms of formulation.

The process of the present invention uses polyurethanes polymers which allow the vaginal ring to have a perfect shape in comparison to vaginal rings made of other polymers such as EVA which once printed shows imperfections due to draining caused by the low melting point of EVA.

Furthermore, polyurethanes due to their high melting point do not need to be cured once the manufacturing of the ring is over. The use of polyurethanes allows also to avoid the storage of the rings at temperatures below 25°C.

Object of the present invention is a method for manufacturing a controlled release vaginal ring using a three-dimensional (3D) printer, said vaginal ring comprising:
(a) a core, said core consisting of a composition comprising one or more thermoplastic polyurethanes and one or more active pharmaceutical ingredients dissolved or dispersed in said core,
(b) a membrane, substantially or completely surrounding said core, said membrane consisting of a composition comprising one or more thermoplastic polyurethanes;
said method comprising the steps:
i) generating a 3D model of a vaginal ring;
ii) preparing a filament or pellets comprising the composition of the core;
iii) preparing a filament or pellets comprising the composition of the membrane;
iv) feeding the filament or pellets comprising the composition of the core and the filament or pellets comprising the composition of the membrane into a different nozzle of a 3D printer;
v) controlling a 3D printer to fabricate the vaginal ring according to the 3D model,
wherein the steps i), ii) and iii) may be carried out in any time sequence.

The vaginal ring has preferably a toroidal coaxial shape.

The thermoplastic polyurethane of the core composition and the thermoplastic polyurethane of the membrane composition are independently selected from the group consisting of thermoplastic polycarbonate - urethane, thermoplastic silicone-polycarbonate-urethane, thermoplastic polyether-urethane, thermoplastic silicone-polyether-urethane and a mixture thereof.

Preferably, the thermoplastic polyurethane of the core composition is different from the thermoplastic polyurethane of the membrane composition.

Most preferably, the thermoplastic polyurethane of the core composition is thermoplastic polyether-urethane and the thermoplastic polyurethane of the membrane composition is thermoplastic silicone-polycarbonate-urethane.

For example, the thermoplastic polyurethane can be Bionate® Thermoplastic Polycarbonate-urethane (PCU), CarboSil® Thermoplastic Silicone-Polycarbonate-urethane (TSPCU), Elasthane™ Thermoplastic Polyether-urethane (TPU, PurSil® Thermoplastic Silicone-Polyether-urethane (TSPU), all commercialized by DSM Biomedical.

In Table the preferred combinations of the polyurethanes used in the core and membrane of the vaginal rings of the invention are shown.

**Table**

| | Combination | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| Bionate® Thermoplastic Polycarbonate-urethane (PCU) | core | core | core | memb. | | | memb. | | | memb. | | |
| CarboSil® Thermoplastic Silicone-Polycarbonate-urethane (TSPCU) | memb. | | | core | core | core | | memb. | | | memb. | |
| Elasthane™ Thermoplastic Polyether-urethane (TPU) | | memb. | | | memb. | | core | core | core | | | memb. |
| PurSil® Thermoplastic Silicone-Polyether-urethane (TSPU) | | | memb. | | | memb. | | | memb. | core | core | core |

| | | | | | | | | | | | | |
|---|---|---|---|---|---|---|---|---|---|---|---|---|
| **Memb.** = **membrane** | | | | | | | | | | | | |

The active principle is selected from progestogen, progestergenic, anti-viral agents and a combination of at least two of said agents.

Preferred progestogen or progestergenic agents are selected from the group consisting of alfatradiol, algestone acetophenide, allylestrenol, altrenogest, androstenedione, bazedoxifene acetate, Buserelin, boldenone undecenoate, cetrorelix acetate, chlormadinone acetate, chlorotrianisene, clomifene citrate, clostebol acetate, conjugated oestrogens, cyclofenil, cyproterone acetate, danazol, degarelix, desogestrel, delmadinone acetate, deslorelin, dienestrol, diethylstilbestrol dipropionate, dienogest, diethylstilbestrol, dienogest, drospirenone, drostanolone propionate, dydrogesterone, elcometrine, estradiol, estradiol dipropionate, estradiol enantate, estradiol hexahydrobenzoate, estradiol, phenylpropionate, estradiol acetate, equilin, estradiol benzoate, estradiol phenylpropionate, estradiol valerate, estradiol cipionate, estradiol, estradiol hemihydrate, norethindrone acetate, estrapronicate, ethinylestradiol, estriol, estrone, ethylestrenol, etonogestrel, flugestone acetate, etynodiol diacetate, fluoxymesterone, gestodene, gestonorone caproate, gestrinone, hexestrol, hydroxyprogesterone caproate, hydroxyestrone diacetate, lynestrenol, medrogestone, medroxyprogesterone acetate, megestrol acetate, melengestrol acetate, methyltestosterone, norethandrolone, norethisterone, nomegestrol acetate, norethisterone acetate, norethisterone enantate, noretynodrel, norgestrel, norgestimate, norgestomet, norgestrienone,normethandrone, oxandrolone, prasterone, progesterone, promegestone, promestriene, proligestone, quinestradol, quinestrol, stanozolol, testosterone, testosterone cipionate, testosterone decanoate,, testosterone undecylate, testosterone phenylpropionate, testosterone propionate, tetrahydrogestrinone, tibolone and trimegestone.

Preferred antiviral agents are selected from the group consisting of abacavir, efavirenz, emtriacitabine, lamivudine, zidovudine, emtriacitabine, rilpivirine, tenofovir disoproxil fumarate, emtricitabine, lamivudine, abacavir, lamivudine, zidovudine, abacavir, lamivudine, zidovudine, emtricitabine, didanosine, stavudine, rilpivirine, etravirine, delavirdine mesylate, efavirenz, nevirapine, tipranavir, indinavir, atazanavir, cobicistat, saquinavir, lopinavir, ritonavir, fosamprenavir, ritonavir, darunavir, cobicistat, darunavir, atazanavir and nelfinavir.

The active principle is present in an amount from 0.001% to 40% w/w of the total ring weight. Preferably the active principle is present in an amount from 0.001 to 25% w/w.

The vaginal ring preferably has an outer diameter ranging from 20 to 70 mm and a thickness ranging from 2 to 15 mm.

Preferably the core has a thickness ranging from 1.4 mm to 15mm and the membrane has a thickness ranging from 50 to 600 µm.

The vaginal ring has an infill ranging from 15% to 100%.

The infill % is the volume of material used in the internal part of a piece, the higher the infill %, the stronger the produced object with better mechanical properties.

The vaginal ring may comprise one or more compartments, each containing a different active principle. In a particular embodiment, each compartment may be surrounded by a membrane having a different composition and/or a different thickness.

Solid Works, Autodesk Fusion 360°, Rhinoceros or any other computer aided design (CAD) or 3D modelling programs can be used for generating a 3D model of a vaginal ring.

A Fused Deposition Modeling (FDM) printer equipped at least with two nozzles can be used for printing the vaginal rings of the invention such as Delta WASP 2040 Turbo2, Delta type 3D printer with Zen dual extruder system

The nozzles of the printer to be used in this specific case have a diameter ranging from 0.2 to 0.6 mm.

The temperature at which the vaginal rings are printed depends on the materials used but in general temperatures range from -20°C to +20°C respectively below/above the melting point of the thermoplastic polymers.

The printing temperatures are in general in the range of 180-210°C.

The print speed may be up to 300 mm/s.

The filament or pellets comprising the composition of the core are prepared by well-known extrusion techniques.

The diameter of each filament to be used is 1.75 mm.

### Example 1

### Etonogestrel and ethinylestradiol vaginal ring

### 1.a) Preparation of the core filament

The polymer used was Elastane TPU 75D produced by DSM

The composition of the core filament was the following:

| **Component** | **Weight for each ring** | **Weight for 100 rings** |
|---|---|---|
| Etonogestrel | 11.700 mg | 1.1700 g |
| Ethinylestradiol | 2.700 mg | 0.2700 g |
| Elastane TPU 75D | 1785.600 mg | 178.500 g |

The components were carefully weighted. Etonogestrel and ethinylestradiol were mixed and added to the pellets of Elastane TPU75D. The obtained mixture was mixed in a V mixer for 20 minutes at 20 rpm.

The resulting mixture was loaded into the hopper of the extruder Noztek Pro Pellet & Powder Filament Extruder. A filament of 1.75 mm ± 0.04 mm diameter to 2mm was obtained using the following extrusion parameters:
Extrusion speed 2m / min
Extrusion temperature: 180°C

The extruded filament was cooled in a water bath at a temperature lower than 12°C.

### 1.b) Preparation of the membrane filament

The polymer used was CarboSil TSPU 20 produced by DSM

A membrane filament having the following composition was prepared:

| **Component** | **Weight for each ring** | **Weight for 100 rings** |
|---|---|---|
| CarboSil TSPU 20 | 200.000 mg | 20.000 g |

The polymer was carefully weighted and loaded into the hopper of the extruder Noztek Pro Pellet & Powder Filament Extruder. A filament of 1.75 mm ± 0.04 mm diameter to 2mm± 0.04 was obtained using the following extrusion parameters:
Extrusion speed 2m / min
Extrusion temperature: 190°C

The extruded filament was cooled in a water bath at a temperature lower than 12°C.

### 1.c) 3D Printing of the vaginal ring

FDM ™ printer, WASP 2040 Turbo2, Delta type with double extruder ZEN system, a 50 micron layer resolution and 0.2 and 0.4 mm diameter nozzles were used.

A 3D model of a coaxial toroidal ring with a diameter of 56.0 mm and a section of 4.0 mm was generated by a CAD 3D program and was imported into the printer program.

A Solid Works, Autodesk Fusion 360°, Rhinoceros or any other computer aided design (CAD) or 3D modelling program was used for the manufacture of vaginal rings.

The two nozzles extruded and deposited the melted layers of material in one single step, creating at the same time the core and the membrane of the vaginal ring.

The vaginal rings were printed at 200°C for both the core and the membrane.

Using the same compositions of the filaments and the procedure reported above several prototypes were manufactured using the parameters listed below:

| Prototype | Membrane layer height | Core layer height | Infill | Print speed | Extrusion temperature | Plate temperature |
|---|---|---|---|---|---|---|
| 1 | 0.1 mm | 0.8mm | 15% | 20 | 200 | 40°C |
| 2 | 0.1 mm | 0.8mm | 50% | 20 | 200 | 40°C |
| 3 | 0.1 mm | 0.8mm | 100% | 20 | 200 | 40°C |

The printed vaginal rings were packed in aluminium / polythene bags.

### Example 2

### 2.a) Preparation of the core filament

The polymer used was PurSil TSPU 20 produced by DSM

The composition of the core filament was the following:

| **Component** | **Weight for each ring** | **Weight for 100 rings** |
|---|---|---|
| Estradiol hemihydrate | 2.000 mg | 0.200 g |
| PurSil TSPU 20 | 1,698.000 mg | 169.800 g |

The components were carefully weighted. Estradiol hemihydrate was added to the pellets of PurSil TSPU 20. The obtained mixture was mixed in a V mixer for 20 minutes at 20 rpm.

The resulting mixture was loaded into the hopper of the extruder Noztek Pro Pellet & Powder Filament Extruder. A filament of 1.75 mm ± 0.04 mm diameter was obtained using the following extrusion parameters:
Extrusion speed 1.5 m/min
Extrusion temperature: 185°C

The extruded filament was cooled in a water bath at a temperature lower than 12°C.

### 2.b) Preparation of the filament of the membrane:

The polymer used was Bionate PCU 80 A commercialized by DSM

A membrane filament having the following composition was prepared:

| **Component** | **Weight for each ring** | **Weight for 100 rings** |
|---|---|---|
| Bionate PCU 80 A | 300.000 mg | 30.000 g |

The polymer was carefully weighted and loaded into the hopper of the extruder Noztek Pro Pellet & Powder Filament Extruder. A filament of 1.75 mm ± 0.04 mm diameter was obtained using the following extrusion parameters:
Extrusion speed 1.5 m/min
Extrusion temperature: 75°C

The extruded filament was cooled in a water bath at a temperature lower than 12°C.

### 2.c) 3D Printing of the vaginal ring

FDM ™ printer, Delta WASP 2040 Turbo2, Delta type with ZEN dual extruder system, a 50 micron layer resolution and 0.2 and 0.4 mm diameter nozzles were used.

A 3D model of a coaxial toroidal ring with a diameter of 56.0 m and a section of 4.0 mm was generated by a CAD 3D program and was imported into the printer program.

A Solid Works, Autodesk Fusion 360° and Rhinoceros computer design program was used for the manufacture of vaginal rings.

The two nozzles extruded and deposited the melted layers of material in one single step, creating at the same time the core and the membrane of the vaginal ring.

The vaginal rings were printed at 200°C for the core and 75°C for the membrane.

Using the same compositions of the filaments and the procedure reported above several prototypes were manufactured using the parameters listed below:

| Prototype | Membrane layer height | Core layer height | Infill | Print speed | Core extrusion temperature | Membrane extrusion temperature | Plate temperature |
|---|---|---|---|---|---|---|---|
| 1 | 0.1 mm | 0.8mm | 15% | 20 | 200°C | 75°C | 40°C |
| 2 | 0.1 mm | 0.8mm | 50% | 20 | 200°C | 75°C | 40°C |
| 3 | 0.1 mm | 0.8mm | 100% | 20 | 200°C | 75°C | 40°C |

The printed vaginal rings were packed in aluminium / polythene bags.

## Claims

1. A method for manufacturing a controlled release vaginal ring using a three-dimensional (3D) printer, said vaginal ring comprising:
a) a core, said core consisting of a composition comprising one or more thermoplastic polyurethanes and one or more active pharmaceutical ingredients dissolved or dispersed in said core,
b) a membrane, substantially or completely surrounding said core, said membrane consisting of a composition comprising one or more thermoplastic polyurethanes;
said method comprising the steps:
i) generating a 3D model of a vaginal ring;
ii) preparing a filament or pellets comprising the composition of the core;
iii) preparing a filament or pellets comprising the composition of the membrane;
iv) feeding the filament or pellets comprising the composition of the core and the filament or pellets comprising the composition of the membrane into a different nozzle of a 3D printer;
v) controlling a 3D printer to fabricate the vaginal ring according to the 3D model,
wherein the steps i), ii) and iii) may be carried out in any time sequence.

2. The method according to claim 1, wherein the thermoplastic polyurethane of the core composition and the thermoplastic polyurethane of the membrane composition are independently selected from the group consisting of thermoplastic polycarbonate - urethane, thermoplastic silicone-polycarbonate-urethane, thermoplastic polyether-urethane, thermoplastic silicone- polyether-urethane and a mixture thereof.

3. The method according to claim 1 or 2, wherein the thermoplastic polyurethane of the core composition is different from the thermoplastic polyurethane of the membrane composition.

4. The method according to claim 3, wherein the thermoplastic polyurethane of the core composition is thermoplastic polyether-urethane and the thermoplastic polyurethane of the membrane composition is thermoplastic silicone-polycarbonate-urethane.

5. The method according any one of the preceding claims, wherein the active principle is selected from progestogen, progestergenic and anti-viral agents or a combination thereof.

6. The method according to claim 5, wherein progestogen or progestergenic agents are selected from the group consisting of alfatradiol, algestone acetophenide, allylestrenol, altrenogest, androstenedione, bazedoxifene acetate, Buserelin, boldenone undecenoate, cetrorelix acetate, chlormadinone acetate, chlorotrianisene, clomifene citrate, clostebol acetate, conjugated oestrogens, cyclofenil, cyproterone acetate, danazol, degarelix, desogestrel, delmadinone acetate, deslorelin, dienestrol, diethylstilbestrol dipropionate, dienogest, diethylstilbestrol, dienogest, drospirenone, drostanolone propionate, dydrogesterone, elcometrine, estradiol, estradiol dipropionate, estradiol enantate, estradiol hexahydrobenzoate, estradiol, phenylpropionate, estradiol acetate, equilin, estradiol benzoate, estradiol phenylpropionate, estradiol valerate, estradiol cipionate, estradiol, estradiol hemihydrate, norethindrone acetate, estrapronicate, ethinylestradiol, estriol, estrone, ethylestrenol, etonogestrel, flugestone acetate, etynodiol diacetate, fluoxymesterone, gestodene, gestonorone caproate, gestrinone, hexestrol, hydroxyprogesterone caproate, hydroxyestrone diacetate, lynestrenol, medrogestone, medroxyprogesterone acetate, megestrol acetate, melengestrol acetate, methyltestosterone, norethandrolone, norethisterone, nomegestrol acetate, norethisterone acetate, norethisterone enantate, noretynodrel, norgestrel, norgestimate, norgestomet, norgestrienone, normethandrone, oxandrolone, prasterone, progesterone, promegestone, promestriene, proligestone, quinestradol, quinestrol, stanozolol, testosterone, testosterone cipionate, testosterone decanoate, testosterone undecylate, testosterone phenylpropionate, testosterone propionate, tetrahydrogestrinone, tibolone and trimegestone.

7. The method according to any one of the preceding claims, wherein the active principle is present in an amount from 0.001% to 40%.

8. The method according to any one of the preceding claims, wherein the vaginal ring has an outer diameter ranging from 20 to 70 mm and a thickness ranging from 2 to 15 mm.

9. The method according to any one of the preceding claims, wherein the core has a thickness ranging from 1.4 mm to 14.950 mm.

10. The method according to any one of the preceding claims, wherein the membrane has a thickness ranging from 50 to 600 µm.

11. The method according to any one of the preceding claims, wherein the vaginal ring has been manufactured with an infill ranging from 15% to 100%.

12. The method according anyone of the preceding claims, wherein the vaginal ring comprises one or more compartments each containing a different active principle.

13. The method according to claim 5, wherein the vaginal ring comprises more compartments each one surrounded by a membrane having a different composition and/or a different thickness.

14. The method according to any one of the preceding claims, wherein the 3D printer is a Fused Deposition Modeling (FDM) printer equipped at least with two nozzles.

15. The method according to any one of the preceding claims, wherein the printing temperature of the core ranges from -20°C to +20°C respectively below/above the melting point of the thermoplastic polymers.

16. The method according to any one of the preceding claims, wherein the printing temperature of the membrane ranges from -20°C to +20°C respectively below/above the melting point of the thermoplastic polymers.

17. A controlled release vaginal ring obtained through the method of claim 1.
